# EUROPÄISCHE PATENTSCHRIFT

(11) **EP 0 596 203 B1**
(45) Veröffentlichungstag und Bekanntmachung des Hinweises auf die Patenterteilung: **17.11.1999**
(21) Anmeldenummer: 93112464.8
(22) Anmeldetag: 04.08.1993
(51) Int. Cl.: A61K 9/20

(54) **Wirkstoffe und wasserlösliche Polymeren enthaltende Zubereitungen in Form fester Teilchen**
Preparations in solid particle form containing active and water-soluble polymers
Préparations sous forme de particules solides contenant des principes actifs et des polymères hydrosolubles

(30) Priorität: 13.08.1992 DE 4226753
(43) Veröffentlichungstag der Anmeldung: 11.05.1994
(73) Patentinhaber: BASF Aktiengesellschaft, 67063 Ludwigshafen (DE)
(72) Erfinder: Grabowski, Sven, Dr., D-6700 Ludwigshafen (DE); Sanner, Axel, Dr., D-6710 Frankenthal (DE); Rosenberg, Joerg, Dr., D-6701 Ellerstadt (DE)

(56) Entgegenhaltungen:
- EP-A- 0 320 051
- EP-A- 0 358 105
- EP-A- 0 440 462
- FR-A- 2 179 044
- GB-A- 2 207 353
- US-A- 4 259 314
- HAGERS HANDBUCH DER PHARM. PRAXIS SEITEN 41-42 (D6).
- KOLLIDON BASF, SEIETN 131-132, 15-17,
- MAKROMOL.CHEM.SUPPL. 7,31, 1984, 31-33

## Beschreibung

Die vorliegende Erfindung betrifft Wirkstoffe enthaltende Zubereitungen in Form fester Teilchen, erhältlich durch innige Vermischung des Wirkstoffes mit einer wasserlöslichen Schmelze aus
a) 10 bis 90 Gew.-% eines wasserlöslichen Polymeren A einer Viskosität Vₐ von 1.000 bis 120.000 mPa·s [cps] und
b) 10 bis 90 Gew.-% eines wasserlöslichen Polieren B einer Viskosität V_{b} von 1 bis 500 mPa·s [cps]
   als Trägersubstanz oder durch Verschmelzen der Wirkstoffe mit den Polymeren A und B, wobei wasserlöslich bedeutet, daß sich bei 20°C in 100 g Wasser mindestens 0,5 g des Polymeren auflösen oder unter Gelbildung lösen, und die Viskositäten Vₐ und V_{b} diejenigen einer 2 gew.-%igen wäßrigen Lösung bei 20°C sind, gemessen nach der Kapillarmethode ASTM D 2363-72 (Europäisches Arzneibuch, Bd. 111, S. 37), und Verarbeitung der Schmelze unter Formgebung zu den Teilchen.

Außerdem betrifft die Erfindung ein Verfahren zur Herstellung dieser Zubereitungen sowie deren Verwendung für die Zwecke der Human- und Veterinärmedizin sowie des Pflanzenschutzes.

Wirkstoffe enthaltende Zubereitungen aus wasserlöslichen Polymeren als Trägersubstanz mit verzögerter Wirkstofffreisetzung sind allgemein bekannt. Die verzögerte Freisetzung wird üblicherweise durch schwer lösliche oder unlösliche Zusatzstoffe wie Copolymerisate aus Methylmethacrylat und Acrylsäure sowie Wachse und Fette erreicht (US-A 3 432 592).

In der EP-A 440 462 sind wirkstoffhaltige Zubereitungen in Form gepreßter Tabletten beschrieben, die Mischungen aus hochviskosen und niederviskosen Hydroxypropylmethylcellulosen als Trägersubstanzen enthalten. Nachteil dieser Zubereitungsformen ist, daß sie hinsichtlich der Freisetzung zu wünschen übrig lassen, da der Wirkstoff kristallin in der polymeren Trägersubstanz eingebettet ist und nach Einnahme nicht vollständig resorbiert werden kann. Außerdem ist eine gleichmäßig verzögerte Freisetzung nur bei bestimmten Mischungsverhältnissen von hoch- zu niederviskoser Hydroxypropylmethylcellulose zu beobachten. Ferner ist die Herstellung dieser Zubereitungsformen technisch sehr aufwendig, da hierbei ein hoher Preßdruck und - für einheitliche Korngrößen - mehrfache Siebvorgänge erforderlich sind.

In der US-A 4 259 314 wird ein Verfahren zur Herstellung von langsam freisetzenden pharmazeutischen Zubereitungen beschrieben, wobei zunächst eine Trockenmischung aus zwei verschiedenen Cellulosederivaten als Trägermaterialien hergestellt wird, und das trockene Trägermaterial nach Vermischen mit einem Wirkstoff zu einer Arzneiform komprimiert wird.

Aus der GB-A 2 207 353 sind pharmazeutische Zubereitungen mit kontrollierter Wirkstofffreisetzung bekannt, die als Bindemittel eine Mischung aus einem pH-abhängigen Alignatsalz und einem pH-unabhängigen Hyrokolloid, beispielsweise Cellulosederivaten oder deren Mischungen enthalten. Aus Bindemitteln und Wirkstoff werden durch Feuchtgranulation Granulate hergestellt, die auf konventionelle Art zu Tabletten gepreßt werden.

In der EP-A 358 105 ist ein Verfahren zur kontinuierlichen Herstellung von festen pharmazeutischen Formen beschrieben, bei dem eine wirkstoffhaltige Schmelze, enthaltend ein oder mehrere Bindemittel extrudiert und verformt wird.

In der FR-A-2179044 wird die Herstellung von Arzneiformen mit hohem Feuchtigkeitsgehalt beschrieben, in denen der Wirkstoff in ein Latexgel, welches wassergefüllte zwischenräume aufweist, eingebettet ist. Diese Arzneiformen können durch Extrusion wasserhaltiger pastöser Massen erhalten werden, welche durch Gefrieren und Trocknen stabilisiert werden.

Der Erfindung lagen daher Wirkstoffe enthaltende Zubereitungen in Form fester Teilchen mit einer verzögerten und gleichmäßigen Wirkstofffreisetzung sowie guter Resorption als Aufgabe zugrunde. Demgemäß wurden die eingangs definierten wirkstoffhaltigen Zubereitungen gefunden.

Weiterhin betrifft die Erfindung ein Verfahren zu deren Herstellung, ihre Darreichungsformen sowie ihre Verwendung für die Zwecke der Human- und Veterinärmedizin sowie des Pflanzenschutzes, in denen die Wirkstoffe verzögert freigesetzt werden sollen.

Die erfindungsgemäßen Zubereitungen enthalten als Trägersubstanz eine wasserlösliche Schmelze aus
a) einem wasserlöslichen Polymeren A einer Viskosität Vₐ von 1.000 bis 120.000 mPa·s [cps], vorzugsweise 3.500 bis 120.000 mPa·s [cps] und
b) einem wasserlöslichen Polymeren B einer Viskosität Vb von l bis 500 mPa·s [cps], vorzugsweise 1 bis 100 mPa·s [cps],
   wobei die Viskositäten Vₐ und V_{b} diejenigen einer 2 Gew.-%igen wäßrigen Lösung bei 20°C sind, gemessen nach der Kapillarmethode ASTM D 2363-72 (Europäisches Arzneibuch, Bd. 111, S. 37), und wobei wasserlöslich bedeutet, daß sich bei 20°C in 100 g Wasser mindestens 0,5 g des Polymeren auflösen oder unter Gelbildung lösen.

Die wasserlösliche Schmelze setzt sich aus
a) 10 bis 90 Gew.-%, vorzugsweise 10 bis 50 Gew.-% eines Polymeren A und
b) l0 bis 90 Gew.-%, vorzugsweise 50 bis 90 Gew.-% eines Polymeren B
   zusammen.

Als wasserlösliche Polymere A mit einer Viskosität V₈ kommen folgende gelbildende Substanzen in Betracht:
- Alkylcellulosen wie Methylcellulose
- Hydroxyalkylcellulosen wie Hydroxymethylcellulose,
- Hydroxyethylcellulose, Hydroxypropylcellulose und
- Hydroxybutylcellulose
- Hydroxyalkylalkylcellulosen wie Hydroxyethylmethylcellulose und Hydroxypropylmethylcellulose
- Carboxyalkylcellulosen wie Carboxymethylcellulose
- Alkalimetallsalze der Carboxyalkylcellulosen wie
- Natriumcarboxymethylcellulose
- Carboxyalkylalkylcellulosen wie Carboxymethylethylcellulose
- Carboxyalkylcelluloseester
- Stärken
- Pektine wie Natriumcarboxymethylamylopektin
- Chitinderivate wie Chitosan
- Polysaccharide wie Alginsäure, ihre Alkalimetall und Ammoniumsalze, Carrageenane, Galaktomannane Traganth, AgarAgar, Gummi arabicum, Guargummi und Xanthangummi
- Polyacrylsäure und ihre Salze
- Polymethacrylsäure und ihre Salze
- Polyvinylalkohol
- Polyvinylpyrrolidon
- Polyalkylenoxide wie Polyethylenoxid und Polypropylenoxid und Copolymere aus Ethylenoxid und Propylenoxid
   sowie Gemische dieser Substanzen.

Als Polymere A sind Methylcellulose, Hydroxypropylcellulose und Hydroxypropylmethylcellulose bevorzugt, insbesondere Hydroxypropylmethylcellulose.

Als wasserlösliche Polymere B mit einer Viskosität V_{b} seien
- Alkylcellulosen wie Methylcellulose
- Hydroxyalkylcellulosen wie Hydroxymethylcellulose,
- Hydroxyethylcellulose, Hydroxypropylcellulose und Hydroxybutylcellulose
- Hydroxyalkylalkylcellulosen wie Hydroxyethylmethylcellulose und Hydroxypropylmethylcellulose
- Polyvinylalkohol
- Polyvinylpyrrolidon
- Polyvinylacetat
- N∼Vinylpyrrolidon∼Vinylacetat∼Copolymere
   sowie deren Gemische genannt.

Als Polymere B sind Hydroxypropylcellulose, Polyvinylpyrrolidon und N-Vinylpyrrolidon-Vinylacetat-Copolymere bevorzugt, insbesondere Hydroxypropylcellulose und Copolymere aus 40 bis 70 Gew.-% N-Vinylpyrrolidon und 30 bis 60 Gew.-% Vinylacetat.

Unter "wasserlöslich" ist zu verstehen, daß sich bei 20°C in 100 g Wasser mindestens 0,5 g, bevorzugt 2 g des Polymeren auflösen bzw. unter Gelbildung lösen.

Als Wirkstoffe kommen neben Vitaminen beispielsweise die in der EP-A 240 904 aufgeführten Verbindungen in Betracht, wobei hier vorzugsweise solche geeignet sind, welche auch in der zitierten Schrift als besonders bevorzugte Wirkstoffe genannt sind. Das erfindungsgemäße Prinzip eignet sich besonders für Zubereitungen mit dem Herzkreislaufmittel Nifedipin (l,4-Dihydro-2,6-dimethyl-4-(2'-nitrophenyl)-3,5-pyridindicarbonsäuredi- methylester) als Wirkstoff.

Darüber hinaus sind unter den Wirkstoffen auch diejenigen zu verstehen, die als bioaktive Substanzen im Bereich des Pflanzenschutzes eingesetzt werden.

Um die erfindungsgemäßen Zubereitungen herzustellen, können die Wirkstoffe entweder direkt als Mischung mit den Polymeren A und B verschmolzen werden oder mit der bereits vorliegenden Polymerschmelze gemischt werden.

In den erfindungsgemäßen Zubereitungen kann der jeweilige Wirkstoff in amorpher Form, nahezu homogen in der Schmelze verteilt, vorliegen, was für eine gleichmäßig verzögerte Freisetzung und gute Resorption von Vorteil ist. üblicherweise bezeichnet man die molekulardispers in einer Polymerschmelze vorliegenden Stoffe auch als "feste Lösungen".

Die Wirkstoffe können - je nach Wirksamkeit und Freisetzungsgeschwindigkeit - in unterschiedlichen Konzentrationen in den erfindungsgemäßen Zubereitungen enthalten sein. So sind Wirkstoffmengen pro Dosiereinheit von ca. 0,l bis 90 Gew.-% - bezogen auf die Schmelze - möglich, vorzugsweise liegen sie bei 0,5 bis 60 Gew.-%.

Im übrigen erfolgt die Vermischung des Wirkstoffes mit der Schmelze in an sich bekannter Weise in Extrudern, vorzugsweise in Ein- und Doppelschneckenextrudern in einem Temperaturbereich zwischen 50 und 200°C. Die Formgebung der wirkstoffhaltigen Schmelze zu den erfindungsgemäßen Zubereitungen kann beispielsweise durch Kalandrierung des Extrudates nach der in der EP-A 240 906 beschriebenen Methode sowie nach dem aus der DE-A 38 30 355 bekannten Verarbeitungsverfahren durch Zerkleinerung des Extrudates mit rotierenden Messern in volumengleiche - noch verformbare - Stücke mit erstarrter Oberfläche und anschließendes Verpressen zu Tabletten in den üblichen Tablettiermaschinen erfolgen.

Man kann die Vermischung des Wirkstoffes mit der Schmelze auch in anderen hierfür geeigneten Apparaturen, mit denen man üblicherweise Kunststoffe verarbeitet, z.B. Kalandern und Spritzgußformen, vornehmen.

Außerdem können die erfindungsgemäßen Zubereitungen die üblichen pharmazeutischen Hilfsstoffe wie Füllmittel, Schmiermittel, Formentrennmittel, Fließregulierungsmittel, Weichmacher, Farbstoffe und Stabilisatoren, in Mengen bis zu ca. 50 Gew.-% bezogen auf die wirkstoffhaltige Schmelze (= 100 %) enthalten.

Als Füllstoffe seien z.B. die Oxide von Magnesium, Aluminium, Silizium und Titan genannt, wobei die Menge an Füllstoff vorzugsweise bei ca. 0,05 bis 50 Gew.-% liegt.

Als Schmier- und Formentrennmittel seien z.B. Calcium-, Magnesium- und Aluminiumstearat sowie Talk und Silicone genannt, wobei die Menge vorzugsweise bei ca. 0,1 bis 3 Gew.-% liegt.

Als Fließregulierungsmittel seien z.B. die Mono-, Di- und Triglyceride der langkettigen Fettsäuren, Wachse und Lecithine genannt, wobei die Menge vorzugsweise bei ca. 0,1 bis 5 Gew.-% liegt.

Als Weichmacher seien z.B. neben niedermolekularen Polyalkylenoxiden wie Polyethylenglykol, Polypropylenglykol und Polyethylenpropylenglykol auch mehrwertige Alkohole wie Propylenglykol, Glycerin, Pentaerythrit und Sorbit sowie Natriumdiethylsulfosuccinat, Mono-, Di- und Triacetat des Glycerin und Polyethylenglykolstearinsäureester genannt. Dabei liegt die Menge an Weichmacher vorzugsweise bei ca. 0,5 bis 15 Gew.-%.

Als Farbstoffe seien z.B. Azofarbstoffe, organische und anorganische Piginente sowie natürliche Färbemittel genannt, wobei die Menge vorzugsweise bei ca. 0,5 bis 3 Gew.-% liegt. Als Stabilisatoren seien beispielsweise Lichtstabilisatoren, Antioxidantien, Radikalfänger und Stabilisatoren gegen mikrobiellen Befall genannt, wobei ihre Menge vorzugsweise bei ca. 0,01 bis 0,05 Gew.-% liegt.

Ferner können saure Zusatzstoffe zur Steuerung der Wirkstofflöslichkeit enthalten sein. Üblich sind z.B. Zitronensäure und Bernsteinsäure.

Es ist möglich, entweder die Hilfsstoffe in die bereits wirkstoffhaltige Polymerschmelze zu mischen oder zusammen als Mischung mit den Wirkstoffen in die Polymerschmelze einzuarbeiten. Ferner können Gemische aus Hilfsstoffen, Wirkstoffen sowie den Polymeren A und B direkt verschmolzen werden. Im allgemeinen ist es üblich, nach der letztgenannten Methode vorzugehen.

Die erfindungsgemäßen wirkstoffhaltigen Zubereitungen werden in Form von Pulvern, Granulaten, Tabletten und Pellets eingesetzt und für die Zwecke der Human- und Veterinärmedizin sowie des Pflanzenschutzes, in denen die Wirkstoffe verzögert freigesetzt werden sollen, verwendet.

Um eine zusätzliche Verzögerung der Wirkstofffreisetzung zu erreichen, können die erfindungsgemäßen Zubereitungen mit Überzugsmaterialien wie Ethylcellulose, Hydroxypropylcellulose, Hydroxypropylmethylcellulose, Celluloseacetatphthalat, Polymethacrylaten und Schellack umhüllt werden. Insbesondere für die Zwecke der Humanmedizin empfiehlt es sich, die Zubereitungen mit Farbüberzügen z.B. aus Titandioxid und aus Buntpigmenten zur Verbesserung des Aussehens zu überziehen. Zur Geschmacksverbesserung eignen sich Überzüge beispielsweise aus Glucose, Saccharose, Xylit und Mannit.

Die neuen Wirkstoffe enthaltenden Zubereitungen in Form fester Teilchen weisen gegenüber denen des Standes der Technik Vorteile auf, indem sie unabhängig von der stofflichen Zusammensetzung die Wirkstoffe gleichmäßig verzögert bei guter Resorption freisetzen, ohne dabei unlösliche Rückstände zu hinterlassen. Die nahezu homogene Verteilung des Wirkstoffes in der polymeren Trägersubstanz in Form einer festen Lösung wird durch einfache Herstellmethoden ohne häufige Siebvorgänge und ohne hohe Preßdrücke erreicht.

### Beispiele l bis 7

Für die Herstellung der Wirkstoffe enthaltenden Zubereitungen wurden die folgenden kommerziell erhältlichen Polymeren verwendet:

### Polymere A:

- A/1: Methylcellulose, Vₐ = 8.000 mPa·s [cps] (Metolos® 5M 8000 der Firma Shin-Etsu Chemical)
- A/2: Hydroxypropylmethylcellulose, ᵥₐ = 4.000 mPa·s [cps] (Methocel® F4 M der Firma Dow Chemical)
- A/3: Hydroxypropylmethylcellulose, Vₐ = 100 000 mPa·s [cps] (Methocel® K100 M der Firma Dow Chemical)

### Polymere B:

- B/l: Copolymerisat aus 60 Gew.-% N-Vinylpyrrolidon (NVP) und 40 Gew.-% Vinylacetat, Vb = 5 mPa·s [cps] (Kollidor® VA 64 der Firma BASF AG)
- B/2: Hydroxypropylmethylcellulose, Vb = 100 mPa·s [cps] (Methocel® Kl00 LV der Firma Dow Chemical)
- B/3: Hydroxypropylcellulose, Vₐ = 2 mPa·s [cps] (Klucel® EF der Firma Hercules)
Als Hilfsstoffe C wurden folgende Substanzen verwendet:
- C/1: Polyethylenglykolstearinsäureester (PEG-9 stearat; Macrogolstearat 400 nach DAB 9) (Cremophor® S9 der Firma BASF AG)
- C/2: Polyethylenglykol, Mw = 4.000 (Lutrol® E 4000 der Firma BASF AG)
- C/3: Polyethylenglykol, Mw = 6.000 (Lutrol® E 6000 der Firma BASF AG)
(Mw = mittleres Molekulargewicht Gewichtsmittel)

Zu den in der Tabelle unter den Beispielen l bis 7 angegebenen Mengen der Polymeren A und B wurden zum Teil noch die Hilfsstoffe C und jeweils der Wirkstoff Nifedipin in den dort genannten Mengen gemischt. Die gesamte Mischung wurde in einem Doppelschneckenextruder hergestellt und danach extrudiert, wobei der Extrudermantel über fünf Temperaturzonen (60, 80, 100, 120 und 130°C) beheizt wurde. Der über die jeweils unterschiedlich beheizte Extruderdüse (130 bis l50°C) austretende Polymerschmelzenstrang wurde in einem Kalander mit einander gegenüberliegenden, nach innen gewölbten Vertiefungen in den Walzenmänteln eingeführt und zu 250 mg-Tabletten formgeprägt.

Die Wirkstofffreisetzung wurde mittels der Rührflügelmethode (paddle-Methode nach USP XXI, US-Arzneibuch) gemessen. Diese In-vitro-Prüfungsmethode dient zur Bestimmung der Lösungsrate von wirkstoffhaltigen Formlingen (z.B. Tabletten).

Hierzu wurden 90O ml einer Phosphatpufferlösung mit dem pH-Wert von 6,8 in einem l l-Gefäß mit Rundboden auf 37°C temperiert. Während des Prüfvorganges befand sich die zu prüfende 250 mg-Tablette auf dem Rundboden des Gefäßes zentral unter dem Rührflügel, der sich mit einer Drehzahl von 100 U/min bewegte. Nach einer Versuchsdauer von jeweils 8 Stunden wurde die Menge an freigesetztem Wirkstoff UV-spektroskopisch bestimmt. In allen Fällen war eine zur Zeit proportionale Wirkstofffreisetzung zu beobachten.

Die Einzelheiten dieser Versuche sowie deren Ergebnisse sind der Tabelle zu entnehmen.

**Tabelle**

| Beispiel | Polymer A (Gew.-%) | Polymer B (Gew.-%) | Hilfsstoff C (Gew.-%) | Nifedipin (Gew.-%) | Temperatur der Düse (°C) | Wirkstofffreisetzung nach 8 h (%) |
|---|---|---|---|---|---|---|
| 1 | 50 A/1 | 50 B1/ | - | 25 | 140 | 64 |
| 2 | 50 A/2 | 50 B/1 | - | 25 | 130 | 59 |
| 3 | 75 A/2 | 25 B/1 | - | 25 | 150 | 27 |
| 4 | 37,5A/3 | 62,5B/1 | - | 25 | 140 | 36 |
| 5 | 10 A/1 | 50 B/2 | - | 25 | 130 | 66 |
| | | 40 B/3 | | | | |
| 6 | 10,5A/1 | 30,5B/2 | 1,3 C/1 | 26,3 | 130 | 56 |
| | | 50 B/3 | 3,9 C/2 | | | |
| 7 | 73,4A/2 | 26,6B/3 | 6,6 C/3 | 26,6 | 130 | 54 |

## Patentansprüche

1. Wirkstoffe enthaltende Zubereitungen in Form fester Teilchen, erhältlich durch innige Vermischung des Wirkstoffes mit einer wasserlöslichen Schmelze aus
a) 10 bis 90 Gew.-% eines wasserlöslichen Polymeren A einer Viskosität Vₐ von 1.000 bis 120.000 mPa·s [cps] und
b) 10 bis 90 Gew.-% eines wasserlöslichen Polymeren B einer Viskosität Vb von l bis 500 mPa·s [cps]
als Trägersubstanz oder durch Verschmelzen der Wirkstoffe als Mischung mit den Polymeren A und B,
wobei wasserlöslich bedeutet, daß sich bei 20°C in 100 g Wasser mindestens 0,5 g des Polymeren auflösen oder unter Gelbildung lösen, und die Viskositäten Vₐ und V_{b} diejenigen einer 2 gew.-%igen wäßrigen Lösung bei 20°C sind, gemessen nach der Kapillarmethode ASTM D 2363-72 (Europäisches Arzneibuch, Bd. 111, S. 37) und Verarbeitung der Schmelze unter Formgebung zu den Teilchen.

2. Wirkstoffe enthaltende Zubereitungen nach Anspruch 1, enthaltend
a) ein wasserlösliches Polymeres A einer Viskosität Vₐ von 3.500 bis 120.000 mPa·s [cps] und
b) ein wasserlösliches Polymeres B einer Viskosität Vb von l bis 100 mPa·s [cps].

3. Wirkstoffe enthaltende Zubereitungen nach Anspruch 1 oder 2, erhältlich unter Verwendung einer wasserlöslichen Schmelze aus
a) 10 bis 50 Gew.-% eines wasserlöslichen Polymeren A und
b) 50 bis 90 Gew.-% eines wasserlöslichen Polieren B.

4. Wirkstoffe enthaltende Zubereitungen nach den Ansprüchen l bis 3, enthaltend Methylcellulose, Hydroxypropylcellulose und/oder Hydroxypropylmethylcellulose als Polymeren A.

5. Wirkstoffe enthaltende Zubereitungen nach den Ansprüchen l bis 4, enthaltend Hydroxypropylcellulose, Polyvinylpyrrolidon und/oder N-Vinylpyrrolidon-Vinylacetat-Copolymere als Polymeren B.

6. Zubereitungen nach den Ansprüchen l bis 5, enthaltend Nifedipin als Wirkstoff.

7. Verfahren zur Herstellung der Wirkstoffe enthaltenden Zubereitungen in Form fester Teilchen gemäß den Ansprüchen 1 bis 6, dadurch gekennzeichnet, daß man die Wirkstoffe als Mischung mit den Polymeren A und B verschmilzt oder den Wirkstoff mit einer wasserlöslichen Schmelze aus den Polymeren A und B innig vermischt und die Schmelze unter Formgebung zu den Teilchen verarbeitet.

8. Wirkstoffe enthaltende Pulver, Granulate, Tabletten und Pellets gemäß dem Anspruch 1 für die Verwendung als Arzneimittel.

9. Verwendung der Wirkstoffe enthaltenden Zubereitungen gemäß dem Anspruch 1 für die Zwecke des Pflanzenschutzes, in denen die Wirkstoffe verzögert freigesetzt werden sollen.

## Claims

1. A composition which contains active substances and is in the form of solid particles, obtainable by intimately mixing the active substance with a water-soluble melt composed of
a) 10 - 90% by weight of a water-soluble polymer A with a viscosity Vₐ of 1,000 - 120,000 mPa·s [cps] and
b) 10 - 90% by weight of a water-soluble polymer B with a viscosity V_{b} of 1 - 500 mPa·s [cps]
as carrier substance or by melting the active substances as a mixture with polymers A and B,
where water-soluble means that at least 0.5 g of the polymer dissolves, or dissolves with gel formation, in 100 g of water at 20°C, and the viscosities Vₐ and V_{b} are those of a 2% by weight aqueous solution at 20°C, measured by the ASTM D 2363-72 capillary method (European Pharmacopoeia, Vol. III, p. 37), and processing the melt with shaping to give the particles.

2. A composition containing active substances as claimed in claim 1, containing
a) a water-soluble polymer A with a viscosity Vₐ of 3,500 - 120,000 mPa·s [cps] and
b) a water-soluble polymer B with a viscosity V_{b} of 1 - 100 mPa·s [cps].

3. A composition containing active substances as claimed in claim 1 or 2, obtainable using a water-soluble melt composed of
a) 10 - 50% by weight of a water-soluble polymer A and
b) 50 - 90% by weight of a water-soluble polymer B.

4. A composition containing active substances as claimed in claims 1 to 3, containing methylcellulose, hydroxypropylcellulose and/or hydroxypropylmethylcellulose as polymer A.

5. A composition containing active substances as claimed in claims 1 to 4, containing hydroxypropylcellulose, polyvinylpyrrolidone and/or N-vinylpyrrolidone/vinyl acetate copolymers as polymer B.

6. A composition as claimed in claims 1 to 5, containing nifedipine as active substance.

7. A process for producing the compositions which contain active substances and are in the form of solid particles as claimed in claims 1 to 6, which comprises melting the active substances as a mixture with polymers A and B or intimately mixing the active substance with a water-soluble melt of polymers A and B, and processing the melt with shaping to give the particles.

8. Powders, granules, tablets and pellets containing active substances as claimed in claim 1 for use as drugs.

9. The use of a composition which contains active substances as claimed in claim 1 for the purposes of crop protection in which delayed release of the active substances is required.

## Revendications

1. Compositions contenant des substances actives, à l'état de particules solides, qu'on obtient par mélange intime de la substance active avec une masse fondue, soluble dans l'eau, consistant en
a) 10 à 90 % en poids d'un polymère soluble dans l'eau A ayant une viscosité Vₐ de 1000 à 120 000 mPa.s et
b) 10 à 90 % en poids d'un polymère soluble dans l'eau B, ayant une viscosité V_{b} de 1 à 500 mPa.s,
qui sert de véhicule, ou par fusion des substances actives mélangées avec les polymères A et B,
l'expression soluble dans l'eau indiquant qu'à 20°C et dans 100 g d'eau, il se dissout au moins 0,5 g du polymère, éventuellement avec formation d'un gel, et les viscosités Vₐ et V_{b} sont celles d'une solution aqueuse à 2 % en poids à 20°C, mesurées par la méthode au capillaire ASTM D 2363/72 (Europäisches Arzneibuch, volume 111, p. 37) et transformation de la masse fondue en particules.

2. Compositions contenant des substances actives selon la revendication 1, contenant
a) un polymère soluble dans l'eau A ayant une viscosité Vₐ de 3500 à 120 00 mPa.s et
b) un polymère soluble dans l'eau B ayant une viscosité V_{b} de 1 à 100 Pa.s.

3. Composition contenant des substances actives selon la revendication 1 ou 2, qu'on obtient en utilisant une masse fondue, soluble dans l'eau, qui consiste en
a) 10 à 50 % en poids d'un polymère soluble dans l'eau A et
b) 50 à 90 % en poids d'un polymère soluble dans l'eau B.

4. Compositions contenant des substances actives selon les revendications 1 à 3, contenant de la méthylcellulose, de l'hydroxypropylcellulose et/ou de l'hydroxypropylméthylcellulose en tant que polymère A.

5. Compositions contenant des substances actives selon les revendications 1 à 4, contenant de l'hydroxypropylcellulose, de la polyvinylpyrrolidone et/ou un copolymère N-vinylpyrrolidone-acétate de vinyle en tant que polymère B.

6. Compositions selon les revendications 1 à 5, contenant du Nifedipin en tant que substance active.

7. Procédé pour la préparation des compositions contenant des substances actives sous la forme de particules solides selon les revendications 1 à 6, caractérisé par le fait que l'on fond les substances actives mélangées avec les polymères A et B ou bien on mélange intimement la substance active avec une masse fondue, soluble dans l'eau, des polymères A et B et on met la masse fondue sous forme de particules.

8. Poudres, granulés, comprimés et pilules contenant des substances actives, selon la revendication 1, pour l'utilisation en tant que médicaments.

9. Utilisation des compositions contenant des substances actives selon la revendication 1 dans des applications de protection des végétaux dans lesquelles les substances actives doivent être libérées avec retard.
